# FASCICULE DE BREVET EUROPEEN

(11) **EP 2 165 704 B1**
(45) Date de publication et mention de la délivrance du brevet: **18.04.2012**
(21) Numéro de dépôt: 09171044.2
(22) Date de dépôt: 23.09.2009
(51) Int. Cl.: A61K 31/137, A61K 45/06, A61P 17/00, A61P 17/08, A61P 17/10, A61P 31/04, A61P 31/10

(54) **Utilisation de l'alvérine dans le traitement des affections cutanées**
Verwendung von Alverin zur Behandlung von Hauterkrankungen
Use of alverine for the treatment of cutaneous affections

(30) Priorité: 23.09.2008 FR 0856382
(43) Date de publication de la demande: 24.03.2010
(73) Titulaire: Fauran, François, 1296 Coppet (CH)
(72) Inventeur: Fauran, François, 1296 Coppet (CH)
(74) Mandataire: Lavé, Michèle

(56) Documents cités:
- EP-A1- 1 269 990
- WO-A2-02/080877
- WO-A2-2008/009860
- FR-A1- 2 798 590
- KRUSZEWSKA H ET AL: "SEARCH OF ANTIMICROBIAL ACITIVITY OF SELECTED NON-ANTIBIOTIC DRUGS" ACTA POLONIAE PHARMACEUTICA, POLISH PHARMACEUTICAL SOCIETY, WARZSAW, PL, vol. 59, 1 janvier 2002 (2002-01-01), pages 436-439, XP008065730 ISSN: 0001-6837

## Description

La présente invention a pour objet une nouvelle utilisation de l'alvérine et de ses sels pharmaceutiquement acceptables, telle que définie dans les revendications.

L'alvérine, ou dipropyline ou fenpropamine, est la N-éthyl-N-(3-phénylpropyl)-benzènepropanamine. Elle est utilisée en médecine depuis plus de 30 ans comme antispasmodique musculotrope principalement dans les manifestations fonctionnelles abdominales en particulier avec météorisme et les colopathies fonctionnelles. Le mécanisme d'action de l'alvérine s'apparente à celui de la papavérine avec une spécificité pour l'intestin et le muscle utérin.

Plusieurs documents de brevet décrivent d'autres utilisations de l'alvérine.

Les demandes de L'Oréal EP1088548 (= FR2798590), WO02080866, WO02080877 et WO2008009860 décrivent l'utilisation de l'alvérine respectivement pour relâcher le tissu cutané, stimuler la croissance des cheveux, en tant qu'agent amincissant ou agent blanchissant de la peau.

Les demandes CEREP EP1641445 et WO2007125074 décrivent l'utilisation de l'alvérine dans le traitement des dépressions ou des dysfonctions sexuelles.

La demande EP-A-1269990 décrit une combinaison d'un composé inhibiteur de l'élastase de la famille des N-acylaminoamides et un agent myorelaxant, qui est choisi parmi un inhibiteur des canaux calciques et/ou un antagoniste des canaux chlore. L'inhibiteur de canaux calciques est préférentiellement l'alvérine et/ou ses sels et/ou ses dérivés et/ou ses analogues et/ou le manganèse et/ou ses sels. La composition est destinée à traiter, entre autres, les désordres du vieillissement et/ou à traiter tous les désordres cutanés associés à une trop grande prolifération des bactéries et/ou des levures cutanées.

L'objet de la présente invention est une nouvelle utilisation de l'alvérine et de ses sels pharmaceutiquement acceptables en tant qu'agent antibactérien et/ou antifongique dans le traitement des affections cutanées induites par Propionibacterium acnes, Malassezia furfur, et/ou Candida albicans.

Ainsi la présente invention concerne l'utilisation de l'alvérine et de ses sels pharmaceutiquement acceptables en tant qu'agent antibactérien et/ou en tant qu'agent antifongique dans le traitement de l'acné, de la dermatite séborrhéique, du pityriasis versicolor et/ou des pellicules.

La prévalence de l'acné est importante, 30% chez l'homme et 24% chez la femme, quel que soit l'âge, avec une prévalence de près de 50% chez l'adolescent.

A son origine se situe un climat hormonal androgénique facilitant la production de sébum au niveau du follicule pilo-sébacé, milieu de culture favorable au développement du Propionibacterium acnes. Cette bactérie anaérobie hydrolyse à l'aide de ses lipases le sébum et les lipides membranaires qui sont tout autant des agents pro-inflammatoires que des facteurs de croissance de la bactérie créant dès lors un système auto-entretenu.

Parmi les traitements locaux comme généraux, les antibiotiques ont une place de choix, il s'agit principalement de cyclines principalement utilisées par voie orale, telles que tétracycline, doxycycline et minocycline, et de macrolides essentiellement utilisés par voie topique, tels que clindamycine et érythromycine.

L'usage des antibiotiques a conduit au développement de résistances particulièrement préoccupantes.

L'étude épidémiologique européenne de ROSS et coll. (Br. J. Dermatol., 2003, 148, p 467-478) portant sur plus de 600 souches de Propionibacterium acnes isolées sur des patients révèle que plus de 80% des souches sont résistantes à un antibiotique, la résistance croisée à l'érythromycine/clindamycine étant la plus commune.

L'étude rétrospective de SIMONART et coll. (Br. J. Dematol., 2005, 153, p 395-403) portant sur l'efficacité de l'érythromycine montre au cours des 20 dernières années une diminution significative de l'efficacité sur les lésions inflammatoires de 60 à 20% pour des solutions topique dosées à 1.5-2 % et appliquées pendant 12 semaines. Corrélativement pendant cette même période les solutions de cet antibiotique disponibles sur le marché qui étaient dans les années 85 dosées à 1,5-2% sont actuellement dosées à 4%.

Il est donc important de trouver de nouveaux produits pouvant être utilisés dans le traitement de l'acné.

Malassezia furfur ou Pityrosporum orbiculare ou Pityrosporum ovale est trouvée communément sur la peau et dans la partie supérieure du tronc où la production de sébum est optimale. La dermatite séborrhéique qui affecte 2 à 5% de la population, le Pityriasis versicolor ainsi que les pellicules sont associées à la présence de cette levure lipophile Malassezia furfur.

Sans vouloir être lié par cette théorie, la demanderesse pense que le fait que l'alvérine soit active sur les microorganismes Propionibacterium acnes, Malassezia furfur et Candida albicans s'explique par le fait que ces trois microorganismes trouvent dans le sébum un milieu favorable à leur développement. En effet, ils possèdent un équipement enzymatique comparable en particulier des lipases et des phospholipases pouvant conduire à la dégradation des structures lipidiques membranaires de l'hôte et passer ainsi du statut de commensal à celui d'agent pathogène. Ceci constitue un trait physiopathologique commun à ces 3 microorganismes et consécutif à leur adaptation à l'hôte (J. XU et coll. PNAS, 2007, 104, n° 47, 18730-18735 et H. BRUGGEMANN et coll. Science, 2004, 305, p. 671-673).

Selon l'invention, on peut utiliser l'alvérine base ou ses sels pharmaceutiquement acceptables, tels que les chlorure, bromure, iodure, nitrate, sulfates, phosphates, formiate, acétate, propionate, oxalate, malonate, succinate, glutarate, malate, tartrate, citrate, pyruvate, acétylacétate et les sels des acides gras saturés ou non en C₄₋₂₈, tels que adipate, palmitate, stéarate et oléate.

On retiendra en particulier le citrate d'alvérine du fait de son usage bien établi en thérapeutique en particulier de sa bonne tolérance et de ses propriétés physico-chimiques : solubilité dans l'eau, stabilité dans les conditions ICH.

Pour le traitement des affections cutanées, l'alvérine et ses sels pharmaceutiquement acceptables peuvent être administrés par voie orale ou par voie topique. L'alvérine base ou ses sels peuvent être mélangés avec des excipients pharmaceutiquement acceptables classiques.

Les compositions pour une administration par voie orale sont par exemple des comprimés, des gélules, des solutions buvables ou des suspensions buvables. Chaque unité de dosage de forme sèche peut contenir 10 à 200 mg de substance active. A titre d'exemple, on peut utiliser des formes orales contenant 30 mg, 60 mg ou 120 mg de citrate d'alvérine.

Les compositions pour une administration par voie topique sont par exemple des lotions, des gels, des crèmes, des shampoings ou des savons dermatologiques. Ces compositions topiques contiennent de préférence des sels de l'alvérine à une concentration de 0,1 à 20% en masse, par exemple du citrate d'alvérine à une concentration de 0,5 à 10% en masse, plus particulièrement de 1 à 3% en masse. Pour le traitement de l'acné, on utilise de préférence des lotions, des gels ou des crèmes. Pour le traitement des affections du cuir chevelu, on utilise de préférence des shampoings.

Dans les gels à usage topique, on utilise de préférence un ou plusieurs agents gélifiants, un ou plusieurs humectants et un ou plusieurs conservateurs.

Les agents gélifiants sont par exemple des carbomères (Carbopol®), polymères de l'acide acrylique, qui permettent la formation de gels transparents, particulièrement indiqués pour le citrate d'alvérine dont la solubilité dans l'eau permet de réaliser des concentrations de 1 à 3% sans utilisation de tensioactif. On peut contrôler les propriétés rhéologiques de ces gels de Carbopol® selon leur nature, leur quantité et le pH final de gélification obtenu par alcalinisation du polymère à des pH supérieurs à 4. Parmi les divers carbomères, on utilise de préférence le carbomère 974P.

En tant qu'humectant, on utilise par exemple la glycérine et/ou le propylène glycol, plus particulièrement la glycérine pour optimiser la tolérance locale.

Les conservateurs sont par exemple les parabènes, plus particulièrement le méthylparabène en association avec le phénoxyéthanol.

Les compositions peuvent contenir en plus de l'alvérine ou de ses sels pharmaceutiquement acceptables une ou plusieurs autres substances actives.

Ces substances actives peuvent être choisies dans le groupe constitué notamment par les agents antibactériens et les agents antifongiques. A titre d'exemple, on peut utiliser l'érythromycine, la clindamycine, le peroxyde de benzoyle, les dérivés imidazolés, tels que le kétoconazole, l'éconazole, le miconazole et le fluconazole, la terbinafine, l'amorolfine, la pyrithione zinc et la ciclopiroxolamine.

Pour le traitement de l'acné, on utilise de préférence le citrate d'alvérine en association avec l'érythromycine, la clindamycine et/ou le peroxyde de benzoyle. On peut également associer le citrate d'alvérine à l'adapalène, agent antiacnéique connu. Pour le traitement de la dermatite séborrhéique, du pityriasis versicolor et/ou des pellicules, on utilise de préférence le citrate d'alvérine en association avec le kétoconazole, l'éconazole, le miconazole, le fluconazole, la terbinafine, l'amorolfine, la pyrithione zinc et/ou la ciclopiroxolamine.

### Exemple 1 : Etude expérimentale de l'activité de l'alvérine sur les affections cutanées

L'efficacité de l'alvérine a été testée sur 3 microorganismes : Propionibacterium acnes, Malassezia furfur et Candida albicans.

Le tableau 1 indique les souches utilisées et les conditions opératoires pour les 3 souches de microorganisme testées.

Le sel d'alvérine utilisé dans les tests est le citrate d'alvérine.

**Tableau 1**

| Microorganisme | Souche | Nombre de microorganismes ensemencés en 10⁵ ufc/ml | Gamme testée en µg/ml |
|---|---|---|---|
| P. acnes | CIP 53.117T | 3,0 | 10⁻³-10⁻²-10⁻¹-1-10 |
| M. furfur | IP 1634.86 | 1,4 | 100-250-500-750-1000 |
| C. albicans | ATCC 10231 | 2,7 | 100-125-150-175-200 |

On a introduit le citrate d'alvérine dans un bouillon nutritif contenant de 2 à 3 10⁵ ufc/ml de microorganisme. Après incubation à 32 ± 2,5°C, on a apprécié la croissance microbienne par l'appariation d'une turbidité et on a effectué un dénombrement des microorganismes dans les tubes demeurés limpides. La concentration minimale inhibitrice (CMI) correspond à la concentration en substance active la plus faible inhibant toute culture visible. Les gammes de concentrations ont été réalisées dans l'eau stérile.

Le tableau 2 indique les valeurs de CMI.

**Tableau 2**

| Microorganisme | Souche | CMI en µg/ml |
|---|---|---|
| Propionibacterium acnes | CIP 53.117T | < 10⁻³ |
| Malassezia furfur | IP 1634.86 | 500 |
| Candida albicans | ATCC 10231 | 100 |

Le fait que la CMI du citrate d'alvérine soit inférieure à 10⁻³ µg/ml démontre l'efficacité de l'alvérine dans le traitement de l'acné.

Bien que la CMI du citrate d'alvérine mesurée sur 2 levures lipophiles (500 µg/ml pour Malassezia furfur et 100 µg/ml pour Candida albicans) soit supérieure à celle rapportée pour les dérivés imidazolés (16 µg/ml pour le miconazole et l'éconazole) et la ciclopiroxolamine (12,5 µg/ml), elle est très inférieure à celle du succinate de lithium sur Malassezia furfur (1,2 10³ à 10⁴ µg/ml). De ce fait, le citrate d'alvérine en raison notamment de son innocuité et de l'absence d'un phénomène de résistance est une molécule d'intérêt dans les affections où ces levures sont associées.

Dans un deuxième essai, on a précisé la CMI du citrate d'alvérine vis-à-vis de Propionibacterium acnes et on a testé l'efficacité du citrate d'alvérine comparativement à 2 antibiotiques utilisés classiquement dans le traitement de l'acné par voie topique, l'érythromycine sous forme de son lactobionate et la clindamycine sous forme de son phosphate.

Le tableau 3 indique les résultats obtenus. La souche utilisée est Propionibacterium acnes CIP 53.117T.

**Tableau 3**

| | Nombre de microorganismes ensemencés (10⁵ ufc/ml) | Gamme testée (µg/ml) | CMI (µg/ml) |
|---|---|---|---|
| Citrate d'alvérine | 3 | 1-2,5-5-7,5 10⁻⁴-10⁻³ | 5 10⁻⁴ |
| Lactobionate d'érythromycine | 2 | 10⁻⁴-10⁻³-10⁻²-10⁻¹ | 10⁻⁴ à 10⁻³ |
| Phosphate de clindamycine | 2 | 10⁻⁴-10⁻³-10⁻²- 10⁻¹ | 10⁻² à 10⁻¹ |

On constate que l'activité bactériostatique du citrate d'alvérine est comparable à celle de l'érythromycine et supérieure à celle de la clindamycine.

### Exemple 2 : Gel topique

On a préparé un gel topique ayant la composition suivante

| | |
|---|---|
| Citrate d'alvérine | 1,00 g |
| Glycérine | 10,00 g |
| Carbomère 974P | 1,00 g |
| Phénoxyéthanol | 0,25 g |
| Méthylparabène | 0,10 g |
| Hydroxyde de sodium (solution à 10%) | qsp pH = 5,4 |
| Eau purifiée | qsp 100 g |

Le citrate d'alvérine, la glycérine, le méthylparabène et le phénoxyéthanol sont dissous dans 90% de la quantité d'eau prescrite. Le carbomère est ensuite ajouté sous agitation; lorsque le mélange est homogène, on procède à la gélification du milieu par ajustement du pH à 5,4 par addition de la solution d'hydroxyde de sodium et du complément de la quantité d'eau purifiée.

Le choix du pH final correspond à celui de la peau, cependant pour des considérations en particulier tenant aux propriétés rhéologiques du gel, il peut être ajusté à des pH supérieurs, par exemple au pH physiologique de 7,35-7,40.

### Exemple 3 : Gélules

On a préparé un mélange pour gélules ayant la composition suivante

| | |
|---|---|
| Citrate d'alvérine | 43,0 g |
| Amidon | 55,5 g |
| Stéarate de magnésium | 1,5 g |

Selon une première variante, on mélange à sec le citrate d'alvérine, l'amidon et le stéarate de magnésium.

Selon une deuxième variante, on granule le citrate d'alvérine et l'amidon avec de l'eau purifiée. Après séchage des granulés, on ajoute le stéarate de magnésium en phase externe.

Le mélange obtenu dans la première ou la deuxième variante permet de réaliser des gélules dosées à 30, 60 ou 120 mg en citrate d'alvérine après répartition de la quantité suffisante du mélange titrant 43% en citrate d'alvérine dans des gélules de taille adaptée, par exemple de taille 3 pour une unité de dosage de 60 mg.

L'alvérine et ses sels pharmaceutiquement acceptables, notamment le citrate d'alvérine, présentent une alternative aux antibiotiques utilisés de manière classique dans le traitement de l'acné.

Enfin, le caractère mixte antibactérien-antifongique du citrate d'alvérine lui permet d'exercer une action significative aussi bien dans les affections cutanées où traditionnellement on utilise des antibiotiques (acné) que dans celles où on utilise des antifongiques (dermatite séborrhéique, pityriasis versicolor, pellicules).

## Revendications

1. Utilisation de l'alvérine ou d'un de ses sels pharmaceutiquement acceptables en tant qu'agent antibactérien et/ou antifongique pour l'obtention d'un médicament pour le traitement des affections cutanées induites par Propionibacterium acnes, Malassezia furfur et/ou Candida albicans.

2. Utilisation selon la revendication 1 pour le traitement de l'acné.

3. Utilisation selon la revendication 1 pour le traitement de la dermatite séborrhéique, du pityriasis versicolor et/ou des pellicules.

4. Utilisation selon l'une quelconque des revendications précédentes, **caractérisée en ce que** le sel pharmaceutiquement acceptable est le citrate d'alvérine.

5. Composition pharmaceutique pour utilisation dans le traitement des affections cutanées induites par Propionibacterium acnes, Malassezia furfur et/ou Candida albicans **caractérisée en ce qu'**elle contient de l'alvérine ou un de ses sels pharmaceutiquement acceptables.

6. Composition pour l'utilisation selon la revendication 5, **caractérisée en ce qu'**elle est à administrer par voie topique.

7. Composition pour l'utilisation selon la revendication 6, **caractérisée en ce qu'**elle est sous forme de lotions, de gels, de crèmes ou de shampoings.

8. Composition pour l'utilisation selon l'une des revendications 5 à 7, **caractérisée en ce que** l'alvérine est utilisée en association avec une ou plusieurs autres substances actives.

9. Composition pour l'utilisation selon la revendication 8, **caractérisée en ce que** la substance active est l'érythromycine, la clindamycine, l'adapalène et/ou le peroxyde de benzoyle.

10. Composition pour l'utilisation selon la revendication 8, **caractérisée en ce que** la substance active est un dérivé imidazolé, tel que le kétoconazole, l'éconazole, le miconazole ou le fluconazole, la terbinafine, l'amorolfine, la pyrithione zinc et/ou la ciclopiroxolamine.

11. Composition pour l'utilisation selon la revendication 5, **caractérisée en ce qu'**elle est à administrer par voie orale.

12. Composition pour utilisation selon la revendication 11, **caractérisée en ce qu'**elle est sous forme de comprimés, de gélules, de solutions buvables ou de suspensions buvables.

13. Composition pour utilisation selon la revendication 11 ou 12, **caractérisée en ce que** l'alvérine est utilisée en association avec une ou plusieurs autres substances actives.

14. Composition pour utilisation selon la revendication 13, **caractérisée en ce que** la substance active est la tétracycline, la doxycycline et/ou la minocycline.

15. Alvérine ou un de ses sels pharmaceutiquement acceptables pour utilisation dans le traitement des affections cutanées induites par Propionibacterium acnes, Malassezia furfur et/ou Candida albicans.

## Claims

1. Use of alverine or of a pharmaceutically acceptable salt thereof as an antibacterial and/or antifungal agent for obtaining a medicament for treating skin conditions caused by *Propionibacterium acnes, Malassezia furfur* and/or *Candida albicans.*

2. Use according to Claim 1, for treating acne.

3. Use according to Claim 1, for treating seborrhoeic dermatitis, pityriasis versicolor and/or dandruff.

4. Use according to any one of the preceding claims, **characterized in that** the pharmaceutically acceptable salt is alverine citrate.

5. Pharmaceutical composition for use in the treatment of skin conditions caused by *Propionibacterium acnes*, *Malassezia furfur* and/or *Candida albicans*, **characterized in that** it contains alverine or a pharmaceutically acceptable salt thereof.

6. Composition for use according to Claim 5, **characterized in that** it is to be administered topically.

7. Composition for use according to Claim 6, **characterized in that** it is in the form of lotions, gels, creams or shampoos.

8. Composition for use according to one of Claims 5 to 7, **characterized in that** the alverine is used in combination with one or more other active substances.

9. Composition for use according to Claim 8, **characterized in that** the active substance is erythromycin, clindamycin, adapalene and/or benzoyl peroxide.

10. Composition for use according to Claim 8, **characterized in that** the active substance is an imidazole derivative, such as ketoconazole, econazole, miconazole or fluconazole, terbinafine, amorolfine, pyrithione zinc and/or ciclopirox olamine.

11. Composition for use according to Claim 5, **characterized in that** it is to be administered orally.

12. Composition for use according to Claim 11, **characterized in that** it is in the form of tablets, gel capsules, oral solutions or oral suspensions.

13. Composition for use according to Claim 11 or 12, **characterized in that** the alverine is used in combination with one or more other active substances.

14. Composition for use according to Claim 13, **characterized in that** the active substance is tetracycline, doxycycline and/or minocycline.

15. Alverine or a pharmaceutically acceptable salt thereof for use in the treatment of skin conditions caused by *Propionibacterium acnes*, *Malassezia furfur* and/or *Candida albicans.*

## Patentansprüche

1. Verwendung von Alverin oder einem pharmazeutisch unbedenklichen Salz davon als antibakterielles und/oder antimykotisches Mittel zum Erhalt eines Arzneimittels zur Behandlung von durch Propionibacterium acnes, Malassezzia furfur und/oder Candida albicans verursachten Hautleiden.

2. Verwendung nach Anspruch 1 zur Behandlung von Akne.

3. Verwendung nach Anspruch 1 zur Behandlung von seborrhoischer Dermatitis, Kleienpilzflechte und/oder Schuppen.

4. Verwendung nach einem der vorhergehenden Ansprüche, **dadurch gekennzeichnet, dass** es sich bei dem pharmazeutisch unbedenklichen Salz um Alverincitrat handelt.

5. Pharmazeutische Zusammensetzung zur Verwendung bei der Behandlung von durch Propionibacterium acnes, Malassezzia furfur und/oder Candida albicans verursachten Hautleiden, **dadurch gekennzeichnet, dass** sie Alverin oder ein pharmazeutisch unbedenkliches Salz davon enthält.

6. Zusammensetzung zur Verwendung nach Anspruch 5, **dadurch gekennzeichnet, dass** sie zur Verabreichung auf topischem Wege vorgesehen ist.

7. Zusammensetzung zur Verwendung nach Anspruch 6, **dadurch gekennzeichnet, dass** sie in Form von Lotionen, Gelen, Cremes oder Shampoos vorliegt.

8. Zusammensetzung zur Verwendung nach einem der Ansprüche 5 bis 7, **dadurch gekennzeichnet, dass** das Alverin in Kombination mit einem oder mehreren anderen Wirkstoffen verwendet wird.

9. Zusammensetzung zur Verwendung nach Anspruch 8, **dadurch gekennzeichnet, dass** es sich bei dem Wirkstoff um Erythromycin, Clindamycin, Adapalen und/oder Benzoylperoxid handelt.

10. Zusammensetzung zur Verwendung nach Anspruch 8, **dadurch gekennzeichnet, dass** es sich bei dem Wirkstoff um ein Imidazolderivat, wie Ketoconazol, Econazol, Miconazol oder Fluconazol, Terbinafin, Amorolfin, Pyrithion-Zink und/oder Ciclopiroxolamin handelt.

11. Zusammensetzung zur Verwendung nach Anspruch 5, **dadurch gekennzeichnet, dass** sie zur Verabreichung auf oralem Wege vorgesehen ist.

12. Zusammensetzung zur Verwendung nach Anspruch 11, **dadurch gekennzeichnet, dass** sie in Form von Tabletten, Gelatinekapseln, Trinklösungen oder Trinksuspensionen vorliegt.

13. Zusammensetzung zur Verwendung nach Anspruch 11 oder 12, **dadurch gekennzeichnet, dass** das Alverin in Kombination mit einem oder mehreren anderen Wirkstoffen verwendet wird.

14. Zusammensetzung zur Verwendung nach Anspruch 13, **dadurch gekennzeichnet, dass** es sich bei dem Wirkstoff um Tetracyclin, Doxycyclin und/oder Minocyclin handelt.

15. Alverin oder ein pharmazeutisch unbedenkliches Salz davon zur Verwendung bei der Behandlung von durch Propionibacterium acnes, Malassezzia furfur und/oder Candida albicans verursachten Hautleiden.
